# EUROPEAN PATENT APPLICATION

(11) **EP 2 283 776 A1**
(43) Date of publication of application: **16.02.2011**
(21) Application number: 09290626.2
(22) Date of filing: 12.08.2009
(51) Int. Cl.: A61B 10/02, A61B 19/00

(54) **Biopsy needle**

(71) Applicant: Coloplast A/S, 3050 Humlebaek (DK)
(72) Inventor: Callede, David, 24200 Sarlat La Caneda (FR)

(57) **Abstract**

The present invention relates to a biopsy needle (1;101) comprising a stylet (2;102) and a cannula (10;110) both extending along an axis A - A. The stylet and the cannula are rotatable relative to each other around the axis A - A between, a locked position wherein relative movement between the stylet and the cannula along the axis A - A is prevented, and an unlocked position wherein relative movement between the stylet and the cannula along the axis A - A is enabled. This facilitates the loading of the biopsy needle into a biopsy gun.

## Description

### TECHNICAL FIELD

The present invention relates to a biopsy needle for use in a biopsy gun which is provided with a locking mechanism to prevent accidental movement of the stylet and cannula relative to each other.

### BACKGROUND

Biopsy needles are used in biopsy guns for taking tissue samples, for example in operations for diagnosing pathological conditions following various examinations of the taken sample and makes it possible to reach, without causing injury, deep-lying organs such as the liver and the kidneys.

Biopsy needle comprises a stylet having a recess at the tip end for receiving tissue sample. Enclosing the stylet there is a cannula which serves to cut the tissue sample away from the surrounding tissue in a longitudinal cutting action wherein the cannula is moved relative to the stylet.

One problem with prior art is that when loading the biopsy needle into the biopsy gun the stylet and cannula moves relative to each other making it difficult to handle. Thus, there is an increased risk that the biopsy needle is damaged, loaded wrongly into the biopsy gun or if unintentionally getting cut by biopsy needle.

Thus, there is a need of facilitating the loading of the biopsy needle into the biopsy gun.

### BRIEF DESCRIPTION

An aspect of the invention is to provide a biopsy needle comprising a stylet extending along an axis A - A between a proximal stylet end and a distal stylet end having, the biopsy needle further comprising a cannula extending along the axis A - A between a proximal cannula end and a distal cannula end at least partly enclosing the stylet, wherein the stylet and the cannula are rotatable relative to each other around the axis A - A between, a locked position wherein relative movement between the stylet and the cannula along the axis A - A is prevented, and an unlocked position wherein relative movement between the stylet and the cannula along the axis A - A is enabled.

This facilitates the loading of the biopsy needle into a biopsy gun as a biopsy needle is provided which is interchangeable between two positions, a locked position which is suitable when the biopsy needle is loaded into the biopsy gun without the user having to be worried whether the stylet and the cannula moves relative to each other and an unlocked position wherein the biopsy needle is ready for use, i.e. the cannula and the stylet is movable relative to each other in the longitudinal direction.

In one embodiment of the biopsy needle as described above a stylet coupling part is arranged at the proximal end of the stylet, and a cannula coupling part is arranged at the proximal end of the cannula, and wherein the stylet coupling part and the cannula coupling part.

This further facilitates handling of the biopsy needle as the use can may manipulate the respective coupling parts instead of the sharp and often relative thin stylet and cannula parts of the biopsy gun.

In one embodiment the rotatable movement of the stylet and the cannula relative to each other between the locked and unlocked position can be provided by forming the cannula coupling part with a tubular coupling member having a tubular compartment for at least partly receiving the stylet coupling part, wherein the tubular compartment is formed with a track which engages with a protrusion formed on the stylet coupling part.

The track thus functions as a limit for the protrusion, thereby controlling the relative movement between the cannula and the core need. Thus, by defining the shape of the track it is possible the pattern and/or extent of movement between the cannula and the stylet.

In one embodiment the track is made up of at least a first and a second leg, the first leg extending circumferential around the axis A - A, and the second leg extending longitudinal along the axis A - A.

It can be understood that when the protrusion travels in the first leg which extends mainly transverse to the longitudinal axis A - A, movement of the cannula and the stylet relative to each other along the axis A - A is limited, however, when the protrusion moves into the second leg the cannula and the stylet is freely movable relative to each other along the axis A - A within the extent of the second leg.

In one embodiment the biopsy needle is provided with a coupling arrangement which allows the protrusion to be moved between the first leg and the second leg via plastic deformation of at least one the stylet coupling part or the cannula coupling part. This provides an added level of security as additional force must be applied in order to move the biopsy needle between its locked and unlocked position.

In another embodiment at least one rib is arranged on an outer surface of either the cannula coupling part or the stylet coupling part, and at least one tab is arranged on an outer surface of the other coupling part.

The rib and tab facilitates the rotational movement between the locked and unlocked position as these function as gripping means. For example a user can easily manipulate the rib and tab relative to each other.

Alternatively a biopsy gun can comprise activation means for at least moving the biopsy needle from the locked position to the unlocked position. Such activation means can for example be provided by a protrusion in the housing of the biopsy gun, e.g. in the lid, which engaged with either the rib or tab when the biopsy gun is closed and thereby rotates the cannula and the stylet relative to each other by engagement of the rib or tab with the protrusion.

The biopsy needle may be provided marketed in a kit comprising a number of biopsy needles as described above and a biopsy gun for use with said biopsy needle. Of course the parts may also be sold separately.

### BRIEF DESCRIPTION OF THE DRAWINGS

- Fig. 1: shows one embodiment of a biopsy needle as described herein,
- Fig. 2: shows another embodiment of a biopsy needle as described herein,
- Fig. 3: shows a top view of the biopsy needle of Fig. 2 along lines III - III,
- Fig. 4 same: shows an alternative position of the parts of the biopsy needle shown from the view as Fig. 3,
- Fig. 5: shows in perspective a stylet of the biopsy needle of Fig. 2 - 4,
- Fig. 6: shows in perspective a cannula of the biopsy needle of Fig. 2 - 4,

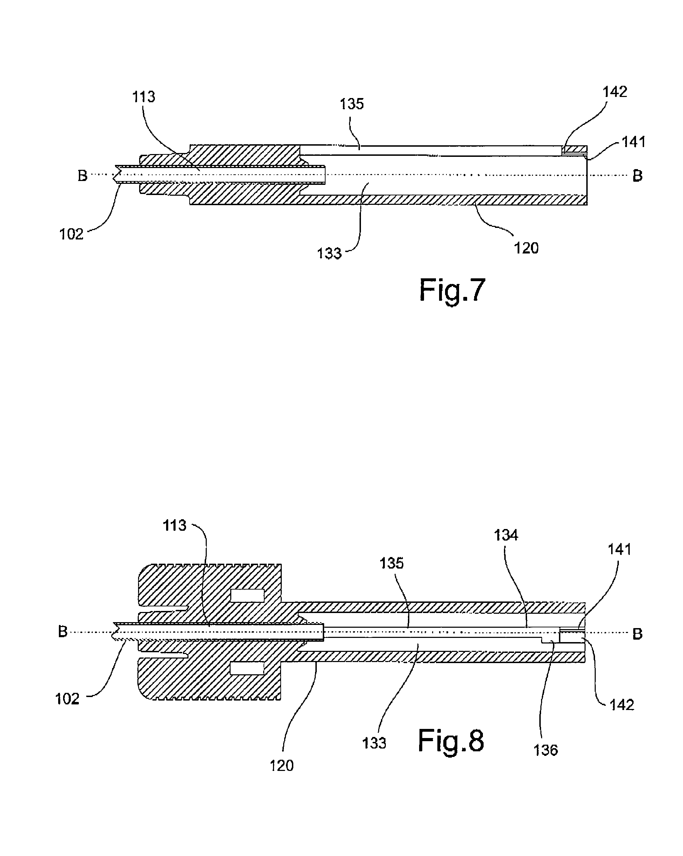

- Fig. 7: shows the cannula of Fig. 6 in section along line VII - VII, and
- Fig. 8: shows the cannula of Fig. 7 in section along line VIII - VIII.

### DETAILED DESCRIPTION

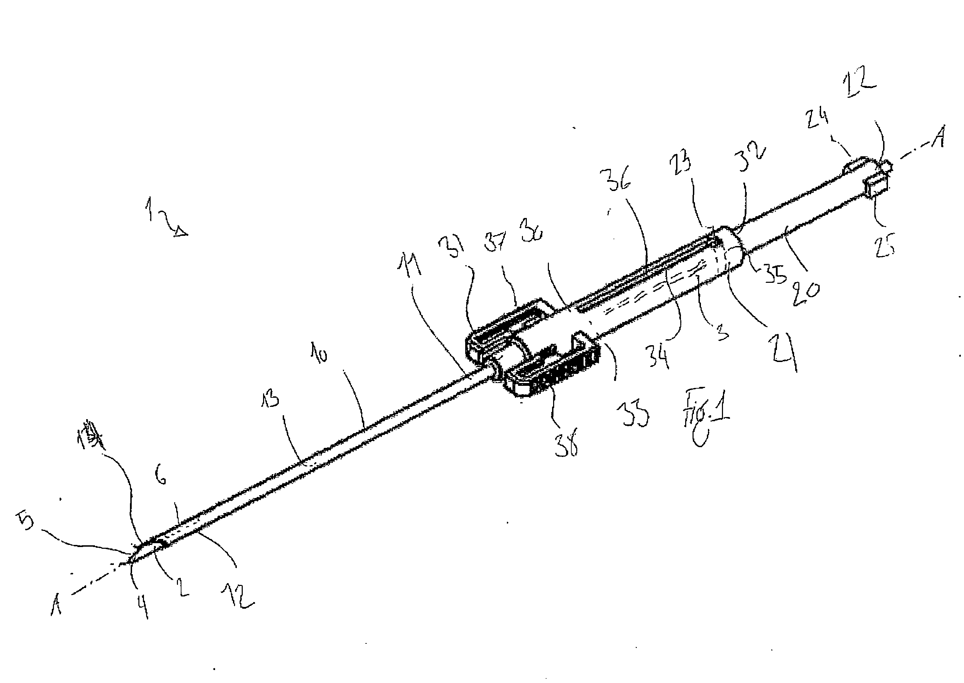

One embodiment of a biopsy needle 1 extending along a longitudinal axis A - A is shown in Fig.1.

In the following it should be understood that when referring to inner and outer surfaces of respective parts this is to be interpreted relative to the surface's orientation relative to the longitudinal axis A - A. Thus, an outer surface will face away from the longitudinal axis, while an inner surface will face towards the longitudinal axis.

The biopsy needle 1 comprises a stylet 2 extending longitudinal between a proximal stylet end 3 and a distal stylet end 4.

The stylet 2 terminates at the distal stylet end 4 in a stylet tip 5, which is sufficiently sharp to penetrate tissue. Further arranged at the distal stylet end 4 is a tissue recess 6 for receiving tissue samples during use of the biopsy needle.

The biopsy needle 1 further comprises a cannula 10 which extends longitudinal between a proximal cannula end 11 and a distal cannula end 12. The cannula 10 is provided a first through going tubular compartment 13 wherein at least a part of the stylet 2 may be received, or in other words the cannula 10 at least partly encloses the stylet 2, allowing the stylet to slide longitudinally along the axis A - A within the cannula 10.

The cannula 10 terminates at distal cannula end 12 in a cannula tip 14, which is sufficiently sharp to penetrate tissue.

A stylet coupling part 20 is attached to the proximal stylet end 3 at a distal stylet coupling part end 21. The stylet coupling part 20 is cylindrically formed and extends longitudinally between the distal stylet coupling part end 21 and a proximal stylet coupling part end 22.

A cannula coupling part 30 is attached to the proximal cannula end 11 at a distal cannula coupling part end 31. The cannula coupling part 30 extends longitudinally between the distal cannula coupling part end 31 and a proximal cannular coupling part end 32; and have a tubular cylindrical shape, defining a second through going tubular compartment 33 which is in communication with the first through going tubular compartment 13 and is capable of at least partly receiving the stylet 2 and the stylet coupling part 20.

A track 34, e.g. in the shape of a groove or a through going slot, is formed on the inner surface of the cannula coupling part 30, i.e. the track 34 opens out into the second through going tubular compartment 33. The track 34 is formed of a first and a second track leg 35, 36. The first track leg 35 extends annularly around the longitudinal axis A - A, in the form a half-circle transversely to the longitudinal axis. The second track leg 36 extends from one end of the first track leg 35 in a direction along the longitudinal axis A - A. Thus, it can be understood that the track is L-shaped, where one of the legs of the L will curve and where the angle between the two legs are approximately 90°. However, other angles between the first and the second track leg can be provided, in particular angles between 45° and 135°.

A coupling protrusion 23 is provided on the outer surface of the stylet coupling part 20, at its distal end 21. The coupling protrusion 23 has a dimension which allows it to be received in the track 34. Thus, when the stylet coupling part 20 is arranged in second through going tubular compartment 33 of the cannula coupling part 30 so that the coupling protrusion 23 extends into the track 34 the movement of the stylet 2 and the cannula 6 will be limited to the shape of the track 34.

In the current embodiment the shape of the track 34 will thus limit relative movement between the stylet 2 and the cannula 6 along the longitudinal axis A - A while the coupling protrusion 23 runs in the first track leg 35, which extends transverse to the longitudinal direction. In other words the biopsy needle can be considered to be in a locked position wherein relative movement between the stylet and the cannula along the axis A - A is prevented.

However, by rotating the stylet 2 and the cannula 6 relative to each other around the axis A - A the coupling protrusion 23 can be moved into the second track leg 36, which extends along the longitudinal direction. Thus, the biopsy needle can be considered to be in an unlocked position wherein relative movement between the stylet 2 and the cannula 6 along the axis A - A is enabled.

The biopsy needle as described above will typically be used in a biopsy gun (not shown), which is suitable for reuse while the biopsy needle will be discarded after each use.

Typical biopsy guns is formed with two slides on which the stylet coupling part 20 and the cannula coupling part 30 are placed respectively. As is commonly know such slides are individually moveable in order to move the stylet 2 and the cannula 10 relative each other.

Protruding from opposite sides of the outer surface of the cannula coupling part 30 there are arranged a first and second rib 37, 38. When these ribs are arranged on the corresponding slide of the biopsy gun it is prevented that the cannula coupling part 30, and thereby the cannula 6, unintentionally rotates around the longitudinal axis.

Protruding from opposite sides of the outer surface of the stylet coupling part 20 there are arranged a first and second tab 24, 25.

When the biopsy needle 1 has been arranged in the biopsy gun, i.e. the stylet coupling part 20 and the cannula coupling part 30 has been placed on the respective slides, the stylet coupling part 20 may be rotated around the longitudinally axis, moving the biopsy needle from its locked position to its unlocked position.

The first and second tab 24, 25 facilitates this rotation as these provide a gripping area on the stylet coupling part 20.

Thus, it can be understood that the biopsy needle can easily be arranged in the biopsy gun without having to keep track of the relative placement of the stylet coupling part and the cannula coupling, as these are locked. When arranged in the biopsy gun the coupling parts are unlocked relative top each other and the biopsy need is thereby ready to use.

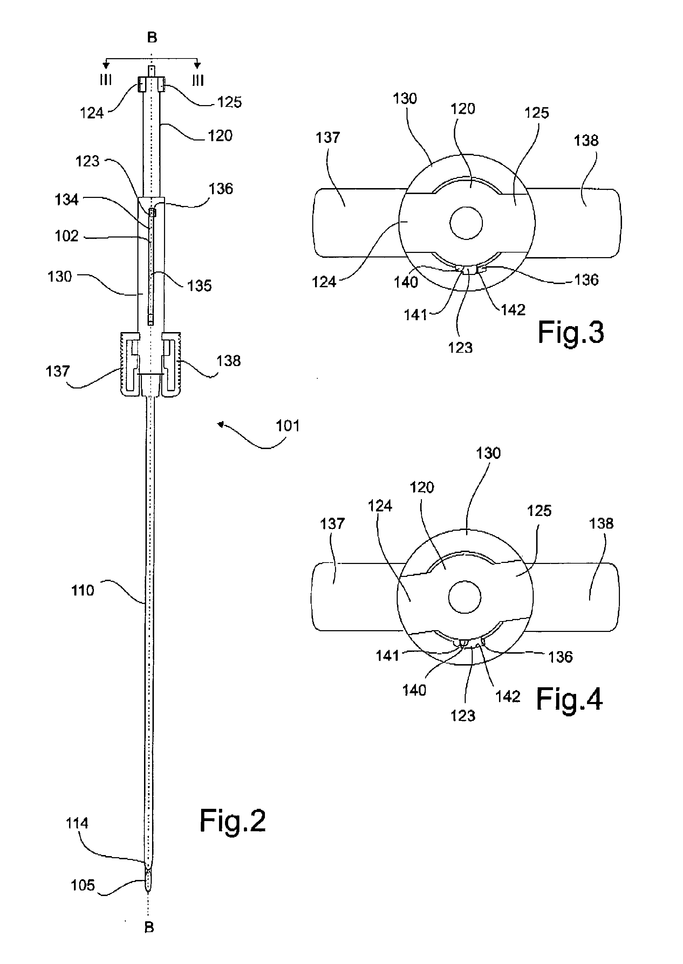

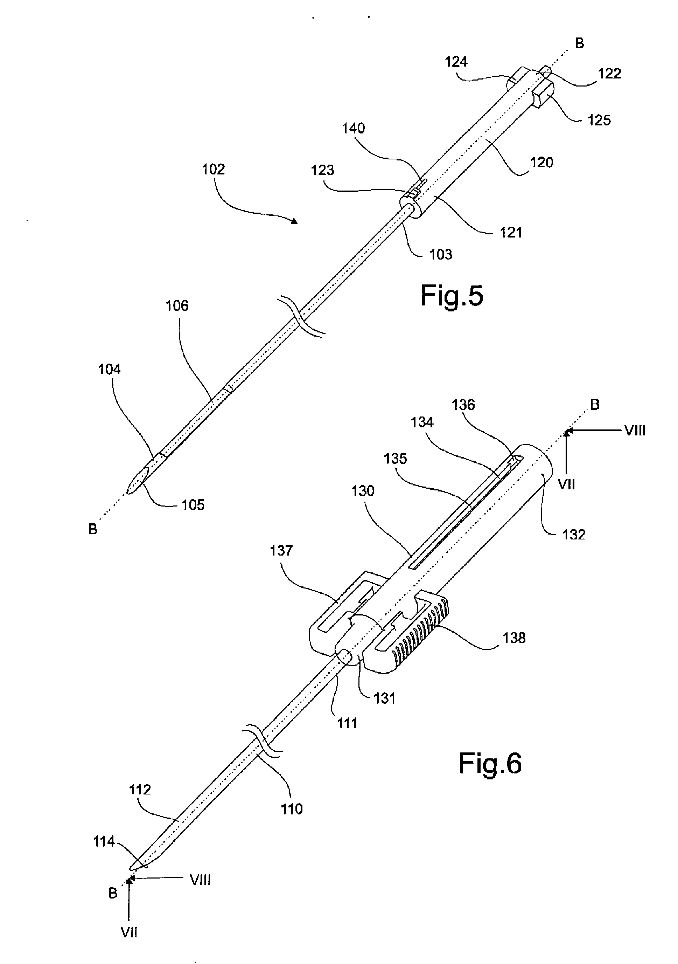

A second embodiment of the biopsy needle 101 extending along a longitudinal axis B - B is shown in figs. 2 - 6.

Similar as with respect to the first embodiment described above it should be understood that when referring to inner and outer surfaces of respective parts this is to be interpreted relative to the surface's orientation relative to the longitudinal axis B - B. Thus, an outer surface will face away from the longitudinal axis, while an inner surface will face towards the longitudinal axis.

The biopsy needle 101 comprises a stylet 102 extending longitudinal between a proximal stylet end 103 and a distal stylet end 104.

The stylet 102 terminates at the distal stylet end 104 in a stylet tip 105, which is sufficiently sharp to penetrate tissue. Further arranged at the distal stylet end 104 is a tissue recess 106 for receiving tissue samples during use of the biopsy needle. The biopsy needle 101 further comprises a cannula 110, which extends longitudinal between a proximal cannula end 111 and a distal cannula end 112. The cannula 110 is provided a first through going tubular compartment 113 wherein at least a part of the stylet 102 may be received, or in other words, the cannula 110 at least partly encloses the stylet 102, allowing the stylet to slide longitudinally along the axis B - B within the cannula 110.

The cannula 110 terminates at distal cannula end 112 in a cannula tip 114, which is sufficiently sharp to penetrate tissue.

A stylet coupling part 120 is attached to the proximal stylet end 103 at a distal stylet coupling part end 121. The stylet coupling part 120 is cylindrically formed and extends longitudinally between the distal stylet coupling part end 121 and a proximal stylet coupling part end 122.

A cannula coupling part 130 is attached to the proximal cannula end 111 at a distal cannula coupling part end 131. The cannula coupling part 130 extends longitudinally between the distal cannula coupling part end 131 and a proximal cannular coupling part end 132; and have a tubular cylindrical shape, defining a second through going tubular compartment 133 which is in communication with the first through going tubular compartment 113 and is capable of at least partly receiving the stylet 102 and the stylet coupling part 120.

A track 134, e.g. in the shape of a groove or a through going slot, is formed on the inner surface of the cannula coupling part 130 so that the track 134 opens out into the second through going tubular compartment 133. The track 134 is formed of a longitudinal track leg 135 and a track recess 136 formed at the distal end of the longitudinal track leg and extending in a direction annularly around the longitudinal axis from the longitudinal axis. Similarly to the first embodiment described above the track 134 can be viewed as L-shaped, where the track recess 136 will slightly curve and where the angle between the two legs are approximately 90°. However, other angles between the first and the second track leg can be provided, in particular angles between 45° and 135°.

A coupling protrusion 123 is provided on the outer surface of the stylet coupling part 120, at its distal end 121. The coupling protrusion 123 has a dimension which allows it to be received in the track 134. Thus, when the stylet coupling part 120 is arranged in second through going tubular compartment 133 of the cannula coupling part 130 so that the coupling protrusion 123 extends into the track 134 the movement of the stylet 102 and the cannula 106 will be limited to the shape of the track 134.

Extending longitudinally and in a proximal direction from the coupling protrusion 123 a first locking rib 140 is provided on the outer surface of the stylet coupling part 120. Extending longitudinal and in a proximal direction from the longitudinal track leg 135 there is provided a surface recess 142 on the inner surface of the cannula coupling part. Said surface recess has a depth which allows the first locking rib 140 to move freely therein when the stylet coupling part 120 is arranged in the cannula coupling part 130. A second locking rib 141 is provided in the surface recess on the inner surface of the cannula coupling part 130.

When the stylet coupling part 120 is arranged in the cannula coupling part 130 the first and second locking rib is so dimensioned that they will abut again each other. However, by applying some force plastic deformation may occur and the two locking ribs can pass each other making it possible to arrange the stylet coupling part and the cannula coupling part in two distinct positions. In itself this principle is generally known and is for example used in coupling arrangements such as in lids for markers and pens and a large number of other applications where at least one part is plastically deformed in order to fit or pass another part. A person skilled in the art would know how to dimension the first and second locking ring depending on the material used in order to achieve the desired force required for the two locking ribs to pass each other.

In the second embodiment a locked position can thus be provided wherein relative movement between the stylet and the cannula along the axis B - B is prevented as the coupling protrusion 123 is retained in the track recess 136 by first and second locking ribs. Then by applying a predetermined amount of torque force, i.e. rotating the stylet coupling part 120 and the cannula coupling part 130 with respect to each other the two locking ribs passes each other and the coupling protrusion is moved out of the track recess 136 and into the longitudinal track leg, thereby placing the biopsy needle in an unlocked position wherein relative movement between the stylet and the cannula along the axis B - B is enabled.

In fig. 3, which is a top view along line III-III in fig. 2, it is shown how the first locking rib 140 and the second locking rib 141 are positioned relative to each other when the biopsy needle is in its unlocked position, i.e. the coupling protrusion 123 is arranged in the longitudinal track leg 135.

In fig. 4 a top view of the biopsy needle corresponding to that of fig. 3 is shown where the biopsy needle is in its locked position. Here it can be seen that the first and second locking rib is arranged opposite of their relative position in the unlocked position whereby the coupling protrusion 123 is at least partly arranged in the track recess 136.

The biopsy needle as described above will typically be used in a biopsy gun (not shown), which is suitable for reuse while the biopsy needle will be discarded after each use.

As described above with respect to the first embodiment, typical biopsy guns are formed with two slides on which the stylet coupling part 120 and the cannula coupling part 130 are placed respectively. As is commonly known such slides are individually moveable in order to move the stylet 102 and the cannula 110 relative each other.

Protruding from opposite sides of the outer surface of the cannula coupling part 130 there are arranged a first and second rib 137, 138. When these ribs are arranged on the corresponding slide of the biopsy gun it is prevented that the cannula coupling part 130, and thereby the cannula 106, unintentionally rotates around the longitudinal axis.

Protruding from opposite sides of the outer surface of the stylet coupling part 120 there are arranged a first and second tab 124, 125.

When the biopsy needle 101 has been arranged in the biopsy gun, i.e. the stylet coupling part 120 and the cannula coupling part 130 has been placed on the respective slides, the stylet coupling part 120 may be rotated around the longitudinally axis, moving the biopsy needle from its locked position to its unlocked position.

The first and second tab 124, 125 facilitates this rotation as these provide a gripping area on the stylet coupling part 120.

Thus, it can be understood that the biopsy needle can easily be arranged in the biopsy gun without having to keep track of the relative placement of the stylet coupling part and the cannula coupling, as these are locked. When arranged in the biopsy gun the coupling parts are unlocked relative top each other and the biopsy need is thereby ready to use.

### REFERENCE NUMBERS

1. first embodiment of the biopsy needle
2. stylet
3. proximal stylet end
4. distal stylet end
5. stylet tip
6. tissue recess
10. cannula
11. proximal cannula end
12. distal cannula end
13. first through going tubular compartment
20. stylet coupling part
21. distal stylet coupling part end
22. proximal stylet coupling part end
23. coupling protrusion
30. cannula coupling part
31. distal cannula coupling part end
32. proximal cannular coupling part end
33. second through going tubular compartment
34. track
35. first track leg
36. second track leg
37. first rib
38. second rib
101. second embodiment of the biopsy needle
102. stylet
103. proximal stylet end
104. distal stylet end
105. stylet tip
106. tissue recess
110. cannula
111. proximal cannula end
112. distal cannula end
113. first through going tubular compartment
114. cannula tip
120. stylet coupling part
121. distal stylet coupling part end
122. proximal stylet coupling part end
124. first tab
125. second tab
130. cannula coupling part
131. distal cannula coupling part end
132. proximal cannula coupling part end
133. second through going tubular compartment
134. track
135. longitudinal track leg
136. track recess
137. first rib
138. second rib
140. first locking rib
141. second locking rib
142. surface recess

## Claims

1. A biopsy needle comprising a stylet extending along an axis A - A between a proximal stylet end and a distal stylet end having, the biopsy needle further comprising a cannula extending along the axis A - A between a proximal cannula end and a distal cannula end at least partly enclosing the stylet, wherein the stylet and the cannula are rotatable relative to each other around the axis A - A between,
- a locked position wherein relative movement between the stylet and the cannula along the axis A - A is prevented, and
- an unlocked position wherein relative movement between the stylet and the cannula along the axis A - A is enabled.

2. A biopsy needle according to claim 1, wherein a stylet coupling part is arranged at the proximal end of the stylet, and a cannula coupling part is arranged at the proximal end of the cannula, and wherein the stylet coupling part and the cannula coupling part

3. A biopsy needle according to claim 2, wherein the cannula coupling part is formed as a tubular coupling member having a tubular compartment for at least partly receiving the stylet coupling part, wherein the tubular compartment is formed with a track which engages with a protrusion formed on the stylet coupling part.

4. A biopsy needle according to claim 3, wherein the track is made up of at least a first and a second leg, the first leg extending circumferential around the axis A - A, and the second leg extending longitudinal along the axis A - A.

5. A biopsy needle according to claim 4, wherein a coupling arrangement is provided which allows the protrusion to be moved between the first leg and the second leg via plastic deformation of at least one the stylet coupling part or the cannula coupling part.

6. A biopsy needle according to any of the claims 1 - 5, wherein at least one rib is arranged on an outer surface of either the cannula coupling part or the stylet coupling part, and at least one tab is arranged on an outer surface of the other coupling part.

7. A biopsy gun for use with a biopsy needle according to anyone of the claims 1- 6, wherein the biopsy gun comprises activation means for at least moving the biopsy needle from the locked position to the unlocked position.

8. A kit comprising a biopsy needle according to anyone of the claims 1 - 6, and a biopsy gun according to claim 7.
